# EUROPÄISCHE PATENTSCHRIFT

(11) **EP 1 348 394 B1**
(45) Veröffentlichungstag und Bekanntmachung des Hinweises auf die Patenterteilung: **22.02.2006**
(21) Anmeldenummer: 03006782.1
(22) Anmeldetag: 26.03.2003
(51) Int. Cl.: A61B 19/00

(54) **Planungs- bzw. Navigationsunterstützung durch generische und erfasste Patientendaten mit zweidimensionaler Anpassung**
Planning or navigation assistance by generic obtained patient data with two-dimensional adaptation
Assistance à la planification ou navigation par des données génériques obtenues de patients avec adaptation bi-dimensionelle

(30) Priorität: 27.03.2002 EP 02007218
(43) Veröffentlichungstag der Anmeldung: 01.10.2003
(73) Patentinhaber: BrainLAB AG, 85551 Kirchheim/Heimstetten (DE)
(72) Erfinder: Vilsmeier, Stefan, 6330 Kufstein (AT); Zeiss, Mario, Minato-ku, Tokyo 106-6005 (JP); Schaffrath, Claus, Dr., 81377 München (DE); Feilkas, Thomas, 85567 Grafing (DE)
(74) Vertreter: Rögner, Jürgen

(56) Entgegenhaltungen:
- WO-A-99/59106
- DE-A- 10 037 491
- DE-A- 10 047 314
- US-A- 5 799 055
- US-A- 6 033 415

## Beschreibung

Die vorliegende Erfindung betrifft ein Verfahren zur computergestützten, medizinischen Navigation bzw. zur präoperativen Behandlungsplanung. Ganz allgemein werden bei solchen Navigationsverfahren mittels einer Positionserfassungseinheit die aktuelle Position eines Patienten oder eines Patientenkörperteils sowie die Positionen von medizinischen Behandlungsgeräten oder behandlungsunterstützenden Geräten erfasst, und die erfassten Positionsdaten werden Körperstrukturdaten zugeordnet, um die Körperstrukturdaten in Zuordnung zu den Positionsdaten gemeinsam im Rahmen der Behandlungsunterstützung zu verwenden. Ein solches Navigationssystem ist beispielsweise in der DE 196 39 615 C2 beschrieben.

Computergestützte, stereotaktische Systeme, die mit Hilfe von Körperstrukturdaten arbeiten, welche aus Tomographie-Erfassungssystemen erhalten wurden, und welche mit Unterstützung von vor Ort erstellten Röntgenbildern arbeiten, sind beispielweise aus der US 4,791,934 und der US 5,799,055 bekannt. Operationsunterstützung durch Röntgenbildaufnahmen wird wiederum beispielsweise in den US-Patenten 5,967,982; 5,772,594 und 5,784,431 erörtert.

Als berührungsfreies, und somit für minimal invasive Eingriffe geeignetes Verfahren ist hierbei die Referenzierung der aktuellen Position der zu operierenden Strukturen mittels präoperativ erstellter dreidimensionaler Patientendaten und vor Ort erstellten Röntgenbildern (Fluoro-CT-Matching) eingeführt worden.

Wenn eine genau arbeitende medizinische Navigation zur Verfügung gestellt werden soll, wird gemäß dem derzeitigen Stand der Technik noch immer unter Zuhilfenahmen von Körperstrukturdaten gearbeitet, die zum Beispiel aus Schichtbild-Erfassungssystemen stammen, wie zum Beispiel Computertomographiegeräten oder Kemspintomographiegeräten. Der zu behandelnde Patient wird dabei vor Ort positionell gegenüber den vorher ermittelten Bilddaten registriert, und Operationsinstrumente werden dann virtuell zu den Bilddaten in gleicher Relation wie zum realen Patienten dargestellt, um die Körperstrukturdaten oder möglicherweise auch Röntgenbilddaten für den Chirurgen im Operationsraum nutzbar zu machen.

Der Nachteil solcher Verfahren, bei denen extra für die Navigation im Rahmen einer Behandlung Schichtbildaufnahmen (CT, MR) oder Röntgenbilder erstellt werden, liegt einerseits in der Strahlenbelastung für den Patienten, die dabei entsteht, und andererseits in dem hohen Kostenaufwand, da solche Geräte sowohl in der Anschaffung als auch in der Wartung und im Betrieb sehr teuer sind.

Es ist versucht worden, Systeme zu entwickeln, die sich ohne vorab separat erfasste Körperstrukturdaten des Patienten einsetzen lassen, beispielsweise auf der Basis von statistischen Modellen von Bilddatensätzen für Körperstrukturen. Jedoch mangelt es solchen Systemen an der erforderlichen Genauigkeit für den jeweils zu behandelnden Patienten.

Die DE 100 37 491 A1 und die WO 99/59106 beschreiben Verfahren zur Bereitstellung von 3D-Informationen unter Zuhilfenahme von Fluoro-Aufnahmen. Der Ausgangspunkt ist bei allen Verfahren das Aufnehmen von Durchleuchtungsaufnahmen des Patienten, bzw. der gewünschten Struktur. Hierbei wird ein Lokalisationssystem benutzt um eine räumliche Information mit den Aufnahmen zu erhalten. Die DE 100 37 491 A1 verwendet initial zwei Fluoro-Bilder um daraus ein grobes 3D-Modell zu rekonstruieren. Weitere Aufnahmen aus unterschiedlichen Winkeln werden verwendet, um das Modell noch genauer zu spezifizieren. Gemäß der WO 99/59106 werden im allgemeinen mindestens drei Fluoro-Aufnahmen des Patienten gemacht. Diese sind anterior-posterior, lateral und anterior-posterior mit zurückgeneigtem Kopf. Zusätzlich zu den Durchleuchtungsaufnahmen werden auch Fotographien des Patienten verwendet. Die Modellanpassung findet bei diesem Stand der Technik aufwendig im dreidimensionalen Raum statt.

Es ist die Aufgabe der vorliegenden Erfindung, ein Verfahren zur computergestützten, medizinischen Navigation bzw. präoperativen Behandlungsplanung bereitzustellen, welches die oben beschriebenen Nachteile des Standes der Technik überwindet. Insbesondere soll die gesundheitsbelastende und kostenintensive Erstellung eines separaten Bilddatensatzes für die Navigation/Behandlungsplanung vermieden werden, wobei trotzdem eine ausreichend genaue Navigation zur Verfügung gestellt werden soll.

Diese Aufgabe wird erfindungsgemäß dadurch den Gegenstand des Patentanspruchs 1 gelöst. Die Unteransprüche definieren bevorzugte Ausführungsformen der Erfindung.

Bei einem erfindungsgemäßen, computergestützten, medizinischen Navigationsverfahren bzw. Behandlungsplanungsverfahren werden vorteilhafterweise Körperstrukturdaten verwendet, die auf der Basis eines generischen Modells erhalten werden, welches durch Verknüpfung mit patientencharakteristischen Erfassungsdaten, insbesondere prä- oder intraoperativ gewonnene Röntgenbilddaten, angepasst worden ist.

Wenn diese Röntgenbilddaten mehr als ein Bild aus unterschiedlichen Projektionsrichtungen umfassen, so stehen diese zweidimensionalen Summationsbilder in der Navigation typischerweise in einer bekannten oder berechenbaren Beziehung zueinander.

Das vorliegende dreidimensionale generische Modell wird hierbei aufgrund dieser bekannten Beziehung den patienteneigenen Röntgenbilder überlagert, und eine Projektion dieses Modells den jeweiligen Röntgenbildern angepasst. Diese Modellanpassung findet somit in Abweichung anderer beschriebener Verfahren, welche zweidimensionale patienteneigene Daten zur Schaffung eines dreidimensionalen Modells heranziehen im zweidimensionalen Raum statt.

Bei dieser Anpassung werden in den patienteneigenen Bilddaten die Projektion anatomischer Landmarken oder Geometrien, wie Knochenkanten oder typische knöcherne Begrenzungen und anatomische Strukturen (zum Beispiel Processus Transversus und Spinosus eines Wirbels, oder der projizierte Verlauf des Pedikels bei einem Wirbelmodell) automatisch oder manuell erkannt. Die Projektion der hinterlegten Modellstrukturen werden diesen zweidimensional erkannten Landmarken daraufhin angepasst.

Diese Anpassung bedingt eine Transformationsvorschrift für das Modell an die patienteneigenen Daten, die es ermöglicht auch die dem Modell hinterlegte Information, beispielsweise einen CT- oder MR-Datensatz, entsprechend zu modifizieren, so dass ein "virtueller" aus Schichtaufnahmen bestehender Datensatz des Patienten für die Navigation Verwendung finden kann.

Dieser Datensatz kann wiederum über eine Projektionsberechnung als Digital Reconstructed Radiography (DRR) dargestellt werden, und zur automatischen oder manuellen Verifikation des Modells mit den patientenspezifischen Daten verglichen werden.

Beim erfindungsgemäßen Verfahren reichen grundsätzlich zwei Durchleuchtungsaufnahmen aus. Es empfiehlt sich diese senkrecht zueinander auszuführen, wie zum Beispiel bei anterior-posterior und lateral Aufnahmen, andere Winkel stellen aber kein Problem dar. Für eine Modellpräzisierung können jedoch auch weitere Durchleuchtungsaufnahmen hinzugezogen werden.

Das generische Modell ist in dem erfindungsgemäßen Verfahren bevorzugt vordefiniert. Es enthält Informationen über die verwendeten Verformungsmodelle und deren Zusammenhänge, sowie Landmarken oder andere Punkten von Interesse die zur Verformung des Modells verwendet werden können. Das Modell kann sowohl aus einem Oberflächenmodell als auch Schichtdatenmodellen oder eine Kombination aus beidem bestehen. Der Vorteil des Oberflächenmodells besteht in der einfachen und schnellen Verformbarkeit die es besonders für Echtzeitanwendungen prädestiniert. Schichtdatenmodelle werden für spezielle Visualisierungen benötigt und bieten erweiterte Informationen über das Volumen einer Struktur.

Um das dreidimensionale Modell in Verbindung mit den Durchleuchtungsbilder zu verwenden, muss es an die Information der Bilder angepasst ("morphed") werden. In der hier beschriebenen Erfindung werden die Landmarken und andere Punkte von Interesse die zur Verformung verwendet werden auf die zweidimensionalen Durchleuchtungsaufnahmen projiziert. Die Verformung des Modell wird durch angleichen der vorgegebenen Landmarken an die Kontur der gewünschten Struktur definiert.

Die Landmarken müssen nicht in mehr als einem Durchleuchtungsbild identifiziert werden. Die Vorraussetzung, das mindestens zwei Bilder benötigt werden, um die Position der Landmarken festzulegen, wie es im Stand der Technik verlangt wird, ist hier nicht gegeben. Es ist ausreichend für die Landmarken, wenn sie nur in einer der Aufnahmen platziert werden. Entgegen dem herkömmlichen Verfahren nach der DE 100 37 491 A1 wird kein grobes Model aus den Projektionen erstellt, da das Modell in dem erfindungsgemäßen Verfahren vordefiniert ist und dadurch für eine genaue Anzeige optimiert werden kann. Die Anzahl der zu i-dentifizierenden Landmarken ist auch wesentlich geringer als im Verfahren nach der WO 99/59106. Für das Beispiel Wirbelsäule werden zum Beispiel maximal 20 Landmarken benötigt.

Die Schichtbilddaten sind zusammen mit dem Modell vordefiniert und es muss dadurch keine weitere präoperative Schichtbilderfassung durchgeführt werden.

Da das verwendete Modell vorzugsweise eine vordefinierte Referenz der zu visualisierenden Struktur darstellt, muss es nur noch an die Durchleuchtungen angepasst werden. Der Vorteil für den Chirurgen ist, das er unabhängig von der Anzahl der Durchleuchtungen und ohne auf aufwendige und teuere Schichtbilddaten (MR) zurückzugreifen zu müssen eine genaue 3D-Darstellung der gewünschten Struktur erhält.

Die Genauigkeit der Registrierung der gewünschten Struktur ist bei Verfahren wie in den Schriften DE 100 37 491 A1 und WO 99/59106 abhängig von der Platzierung der Landmarken, bzw. der Erkennung der Kontur im Durchleuchtungsbild. In dem erfindungsgemäß vorgestellten Verfahren kann dieser Fehler noch minimiert werden. Dazu werden die mit dem generischen Modell vordefinierten Durchleuchtungsdaten verwendet. Die Verformungsvorschrift, generiert aus den Informationen der Durchleuchtungsbilder, wird dazu auf die Schichtdaten angewendet. Dadurch erhält man einen an den Patienten angepassten Schichtbilddatensatz. Unter Verwendung eines Fluoro-To-CT Matching Algorithmus kann der deformierte Schichtbilddatensatz noch zusätzlich registriert werden. Dadurch werden Ungenauigkeiten, die durch die manuelle oder automatische Platzierung der Landmarken entstehen können, minimiert. Mit Hilfe dieses Schrittes, kann die Genauigkeit des Verfahrens noch wesentlich gesteigert und auch die Sicherheit verbessert werden. Die Genauigkeit des Fluoro-To-CT Matching Verfahrens wurde in mehren Studien nachgewiesen und die Ergebnisse dieses Algorithmus sind leicht zu verifizieren.

Wenn im Weiteren von "Navigation" die Rede ist, so soll dieser Begriff auch grundsätzlich die präoperative Behandlungsplanung mit umfassen, im Rahmen der ein Chirurg vorab eine ideale Vorgehensweise für eine Behandlung bestimmt, also beispielsweise die ideale Position eines Implantats manuell bestimmt und festlegt.

Die Vorteile der vorliegenden Erfindung beruhen unter anderem darauf, dass es mit der Verwendung eines an den Patienten angepassten generischen Modells nicht mehr notwendig ist, für die Behandlung, bei der eine medizinische Navigation bereltgestellt werden soll, einen separaten Datensatz für die Körperstruktur zu erstellen. Einerseits erspart man dem Patienten dabei Strahlungsbelastungen, und die Kosten für solche Datensatzerstellungen (zum Beispiel durch Tomographie), können eingespart werden, während andererseits durch die Verknüpfung der generischen Körperstrukturdaten mit patientencharakteristischen Erfassungsdaten ein Datensatz zur Verfügung gestellt wird, mit dessen Hilfe eine sehr genaue medizinische Navigation ermöglicht wird. Das generische Modell, das eine Art allgemeingültiges Modell für die betreffende Körperstruktur sein kann, für das sämtliche relevante Daten vorliegen, umfasst dabei zwar nicht Daten, die speziell auf den betreffenden Patienten zugeschnitten sind, jedoch umfasst es ausreichend Anatomie- bzw. Körperstrukturdaten, um nach der Anpassung mit Hilfe patientencharakteristischer Erfassungsdaten eine ausreichend genaue Basis für eine medizinische Navigation zur Verfügung stellen zu können.

Es ist im Rahmen der vorliegenden Erfindung möglich, die Körperstrukturdaten in der Form eines Bilddatensatzes bereitzustellen, insbesondere als Schichtbilderfassungs-Datensatz.

Hierbei werden nicht, wie bei Verfahren gemäß dem Stand der Technik separate Bilderfassungs-Datensätze erzeugt, sondern das generische Modell selbst wird schon in Form eines Bilddatensatzes bereitgestellt, der dann noch an den jeweiligen Patienten angepasst wird, um einen für den Patienten geltenden Bilddatensatz zu erhalten. Diese Anpassung erfolgt anhand einer Überlagerung beispielsweise patientenspezifischen Röntgenbilder, welche ein zweidimensionales Summationsbild aus einer definierten Projektionsrichtung darstellen, und einer Projektion des dreidimensionalen generischen Modells auf dieses Summationsbild.

Die Anpassung der Projektion des Modells auf den patientenspezifischen Daten erfolgt im impliziten Wissen um die dreidimensionale modellspezifische Abhängigkeit von anatomischen Landmarken und Strukturen. Somit kann eine manuelle oder automatische Formanpassung zwischen dem Modell und den Patientendaten je nach Struktur einer Verschiebung, Verformung oder Rotation des Modells, oder einer Kombination hieraus entsprechen.

Die so für das Modell gewonnene Verformungs-, Verschiebungs- und Rotationsvorschrift kann dann auf die dem Modell hinterliegende Information angewendet werden, um einen dreidimensionalen Bilddatensatz zu erzeugen oder einen schon vorhandenen Bilddatensatz mit Hilfe dieser Vorschrift zu verformen.

Mit diesem Bilddatensatz kann dann ebenso gearbeitet werden, wie mit einem, der kostenintensiv und unter Bestrahlungsbelastung präoperativ von dem Patienten erstellt worden ist. Es ist beispielsweise denkbar, ein generisches Modell zu verwenden, das eine typische bzw. durchschnittliche Körperstruktur umfasst, beispielsweise eine einfache modellhafte Darstellung eines Wirbelkörpers, eines Oberarm-, Unterarm-, Oberschenkel-, Unterschenkel- oder Beckenknochens, oder einer sonstigen knöchernen Körperstruktur.

Das generische Modell kann auch ein statistisches Modell der Körperstruktur umfassen, insbesondere basierend auf statistischen Auswertungen einer unbestimmten Anzahl von Bilddatensätzen, zum Beispiel von realen Wirbelkörper-Bilddatensätzen.

Ferner besteht die Möglichkeit, das generische Modell gleich als eine Art Modell-Paket für eine Vielzahl von Körperstrukturen gleicher Art bereitzustellen. In einem solchen Fall ist es bei der Anpassung des Modells möglich, sich aus der Vielzahl der Modelle im Paket dasjenige auszusuchen, welches am besten zu den patientencharakteristischen Erfassungsdaten passt, so dass nur eine geringere computergestützte Anpassung des Modells vorgenommen werden muss.

Das generische Modell kann im Rahmen der vorliegenden Erfindung einem zwei- oder dreidimensionalen Datensatz einer Körperstruktur umfassen, insbesondere auch ein geometrisches Modell. Mit anderen Worten kann es sich beim generischen Modell sowohl um dreidimensionale (zum Beispiel Wirbelkörpermodell) als auch um zweidimensionale Daten (zum Beispiel virtuelle Röntgenbilder) oder aber auch um ein Modell in Form von geometrischen Daten handeln. Diese Daten können beispielsweise Winkel und/oder Trajektorieninformationen sein, die für den Arzt dargestellt werden und ihm beispielsweise die Idealposition eines Implantates anzeigen.

Im Folgenden werden verschiedene Arten von patientencharakteristischen Daten aufgezeigt, wie sie zur Anpassung des generischen Modells verwendet werden können. Es ist immer auch möglich, Kombinationen solcher Daten, die im Weiteren als Diagnosedaten bezeichnet werden, hierfür einzusetzen.

Die patientencharakteristischen Daten können Röntgenbilddaten sein, und zwar solche aus vorab oder während der Behandlung erstellten Röntgenbildern, insbesondere zwei- oder multiplanaren Röntgenbildern. Ein Beispielsfall wäre derjenige, wo für den Patienten schon Röntgenbilder vorliegen, die im Rahmen zeitlich zurückliegender Untersuchungen erstellt worden sind. Daten über Körperstrukturen aus diesen "alten" Röntgenbildern eignen sich insbesondere, wenn Formabweichungen gegenüber dem generischen Modell einberechnet werden sollen.

Es ist aber auch möglich, noch während der Behandlung einzelne Röntgenbilder des Patienten zu erstellen und diese Informationen dann in die Anpassung des generischen Modells einfließen zu lassen. Der Vorteil gegenüber der herkömmlichen "Röntgennavigation" liegt dabei darin, dass nicht eine große Vielzahl von Röntgenbildern erstellt werden muss, wie sie bei der Navigation auf der Basis von Röntgenbildern verwendet wird; es genügt die Erstellung nur eines oder sehr weniger Röntgenbilder zur Anpassung des generischen Modells, die sich außerdem auf ein sehr kleinen Körperabschnitt begrenzen kann. Damit wird die Strahlenbelastung gegenüber herkömmlicher Röntgennavigation stark verringert.

Obiges gilt in gleicher Weise auch für Computertomographie- oder Kernspintomographie-Bilddaten. Es können solche verwendet werden, die aus sehr viel früher erstellten Tomographieerfassungen hervorgehen, deren Information jedoch genügt, um eine geeignete Anpassung des generischen Modells vorzunehmen.

Die diagnostischen Bilddaten können außerdem digital rekonstruierte Röntgenbilddaten (DRRs = digitally reconstructed radiographs) sein, die zum Beispiel aus schon vorhandenen Tomographie-Bilddatensätzen erstellt werden können, ohne dass der Patient nochmals einer Röntgenaufnahme unterzogen werden muss.

Es ist aber nicht unbedingt nötig, in dieser Weise komplizierte, patientencharakteristische Erfassungsdaten bzw. Diagnosedaten zu verwenden, um eine ausreichende Anpassung des generischen Modells vornehmen zu können. Es kann durchaus genügen, akquirierte Punkt-Positionsinformationen der Patienten-Körperstruktur, insbesondere von natürlichen oder künstlichen Landmarken, zu benutzen. Bei den patientencharakteristischen Diagnosedaten kann es sich dann beispielsweise nur um den Abstand zwischen zwei Landmarken (zum Beispiel Knochenfortsätzen) handeln, welcher schon genügend Informationen darüber geben kann, in welcher Weise das generische Modell zu restrukturieren ist. Ebenso können als Basis hierfür Daten über Größe, Gewicht oder Körperabschnitts- bzw. Gliedmaßenlängen des Patienten zum Einsatz kommen.

Die Anpassung des generischen Modells kann im Rahmen der Erfindung durch eine oder mehrere der folgenden Methoden erfolgen:
- manuelle Anpassung mit Hilfe einer Bilddarstellungsunterstützung, insbesondere durch das Versetzen von Punkten und Landmarken oder das Verschieben, Verdrehen, Dehnen, oder Komprimieren des generischen Modells auf einer Bildschirmausgabe mittels Benutzerinterface-Einrichtungen,
- automatische Bildfusionsverfahren, die insbesondere auf der automatischen Erkennung bestimmter anatomischer Merkmale basieren,
- Bilddaten des generischen Modells, insbesondere digital rekonstruierte Röntgenbilder, und solche aus Computertomographie- oder Kernspintomographie-Bilddatensätzen werden in Deckung gebracht bzw. fusioniert.

Die Fusion des generischen Modells mit diagnostischen Verfahren kann also entweder automatisch erfolgen, zum Beispiel durch automatische Erkennung bestimmter anatomischer Merkmale, die für die Fusion entscheidend sind, oder aber manuell, zum Beispiel durch Verschieben, Verdrehen, Stretchen/Verziehen . Wenn eine Fusion des generischen Modells mit echten Patienteninformationen mit Hilfe der Akquirierung einer unbestimmten Anzahl von Punktinformationen am Patienten erfolgt (Landmarken), ist es möglich, ein sogenanntes Surface-Matching-Verfahren, also ein computergestütztes Bildanpassungsverfahren zu verwenden, um die Fusion der Bilddaten durchzuführen. Eine Kombination der Diagnosedatenerfassung sowie der Anpassung des generischen Modells aus verschiedenen oben beschriebenen Methoden wird gemäß einer Ausführungsform der Erfindung so durchgeführt, dass neben den diagnostischen Daten (zum Beispiel intraoperativ akquirierte Röntgenbilder) noch zusätzliche Punkte am Patienten in Form von Landmarken oder zufällig akquirierten Punkten aufgenommen und dazu verwendet werden, die Position des Modells oder dessen Form selbst noch genauer zu erfassen und einzurichten, um damit eine genauere Navigation zu ermöglichen.

Auch die Registrierung einer Hüft-Oberschenkel-Konstellation (Pelvis/Femur) kann mittels registrierter Röntgenbilder und generischem Modell erfolgen. Die Registrierung der Hüfte wäre dabei beispielsweise durch Zuordnung von Landmarken zwischen dem generischen Modell und einem oder mehreren Röntgenbildern (Fluoroskopie, zum Beispiel in einem Abstand von 30°) durch mathematische Kopplung möglich.

Allgemein gesagt, können bei dem erfindungsgemäßen Verfahren die Positionsdaten, die bei der Ermittlung der patientencharakteristischen Erfassungsdaten erhalten werden, insbesondere durch die Akquirierung von Landmarkenpositionen oder durch im Navigationssystem registrierte Röntgenbildaufnahmen, dazu verwendet werden, die Registrierung der angepassten Körperstrukturdaten im Navigationssystem vorzunehmen und Behandlungsgeräte bzw. bchandlungsunterstützende Geräte in Registrierung zur angepassten Körperstruktur bildlich darzustellen oder einzusetzen. Mit anderen Worten wird der Schritt der Erfassung der Diagnosedaten dabei auch gleichzeitig dazu genutzt, die Registrierung von Patient und angepasstem generischen Modell für die Navigation vorzunehmen. Sobald die Daten des Modells mit registrierten Daten, d. h. Daten, die im Raum eindeutig in ihrer Position bestimmt sind, zum Beispiel registrierte Fluoroskopiebilder einer Röntgennavigationssoftware fusioniert werden, oder die Daten des Modells mit Landmarken oder einer Kombination beider Verfahren registriert werden, können diese für die computerassistierte Chirurgie und zum Beispiel für minimal invasive Operationen eingesetzt werden, in dem Instrumente oder Implantate in Relation zu einem fusionierten Modell dargestellt werden.

Das Verfahren gemäß der vorliegenden Erfindung lässt sich sowohl für die Unterstützung chirurgischer Eingriffe anwenden, wobei dem Chirurgen Navigationshilfen auf Bildschirmen bereitgestellt werden, als auch im Rahmen der Strahlentherapie bzw. Radiochirurgie. Die Navigation kann auf einem optischen Tracking oder auf einem magnetischen Tracking beruhen.

Die vorliegende Erfindung umfasst ferner ein Programm, das, wenn es auf einem Computer läuft oder in einen Computer geladen ist, den Computer veranlasst eines der oben beschriebenen Verfahren durchzuführen, sowie ein Computerprogramm-Speichermedium, das ein solches Programm aufweist.

Zusammenfassend ist zur obigen Erfindung noch festzustellen, dass sie die Strahlungsbelastung reduziert und die Kosten für Schichtbild-Aufnahmeverfahren eliminiert und zumindest minimiert und gegenüber der reinen Röntgennavigation auch noch den Vorteil hat, dass sie dem Chirurgen eine dreidimensionale Navigation und Orientierung ermöglicht. Weitere Vorteile liegen noch darin, dass die Schritte, die zu einer Patientenregistrierung führen, wesentlich unkomplizierter sind und das erfindungsgemäße Verfahren mit wenigen manuellen Schritten zur Patientenregistrierung führen kann. Aufwändige diagnostische Untersuchungen werden vereinfacht und es kann beispielsweise auch die Akquirierung von Punkten/Landmarken am Patienten für die Registrierung überflüssig gemacht werden, wenn bereits kalibrierte Daten (zum Beispiel registrierte Röntgendaten) eingesetzt werden und zusätzliche nützliche Informationen durch Fusion mit dem generischen Modell (zum Beispiel Umwandlung mehrerer zweidimensionaler Informationen zu echten dreidimensionalen Informationen) gewonnen werden.

Die automatische Darstellung der Idealposition von Implantaten oder Instrumenten wird erfindungsgemäß somit mit geringem Aufwand möglich, so dass die Eingriffe schneller, sicherer und weniger invasiv durchführbar sind.

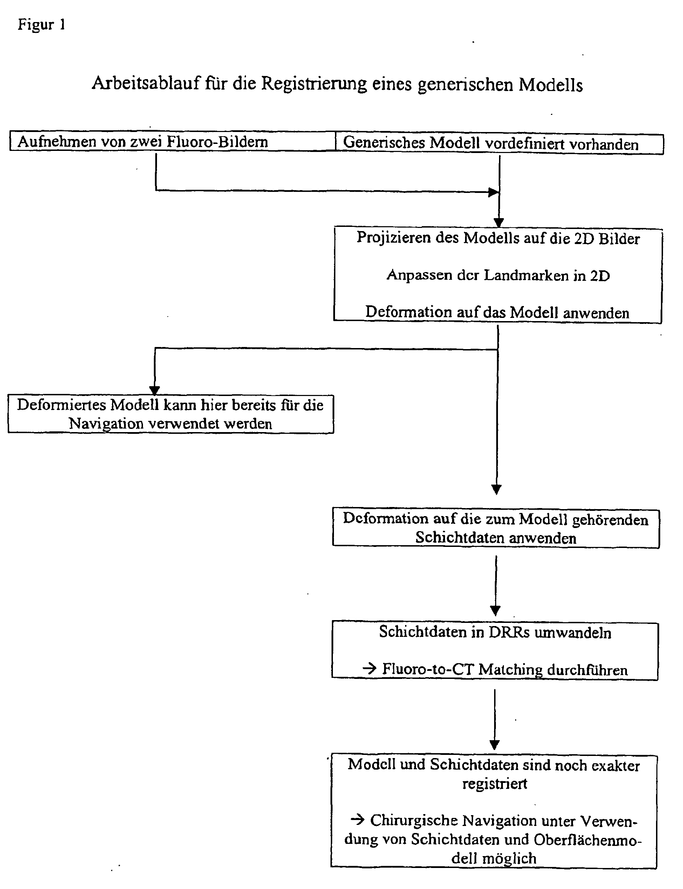

Die beiliegende Figur 1 zeigt ein selbsterklärendes Ablaufdiagramm für die Registrierung und Anpassung eines generischen Modells gemäß der Erfindung.

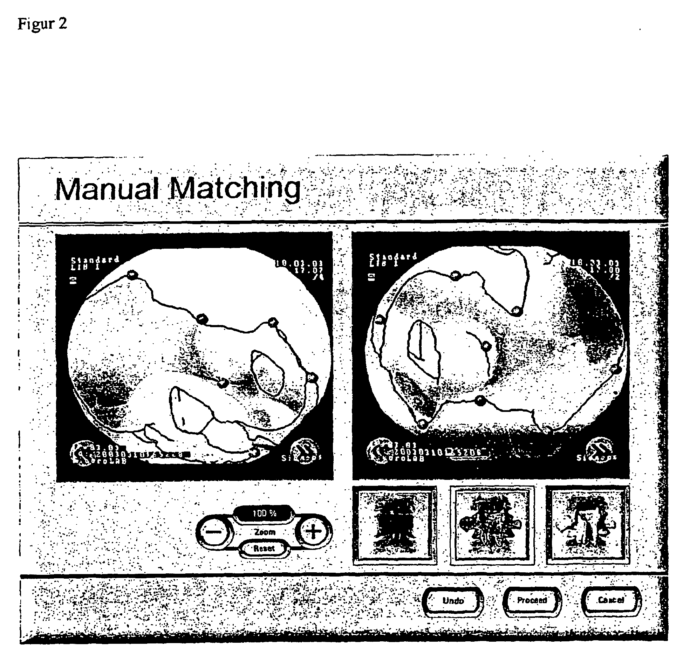

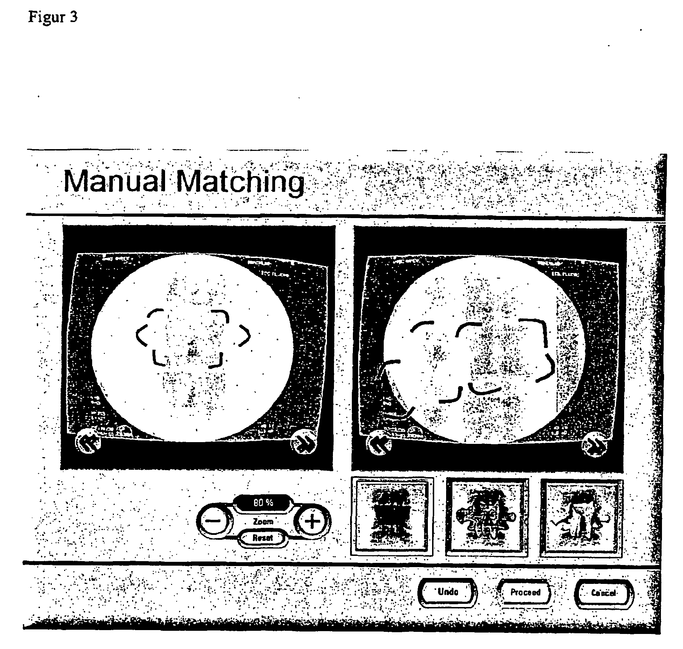

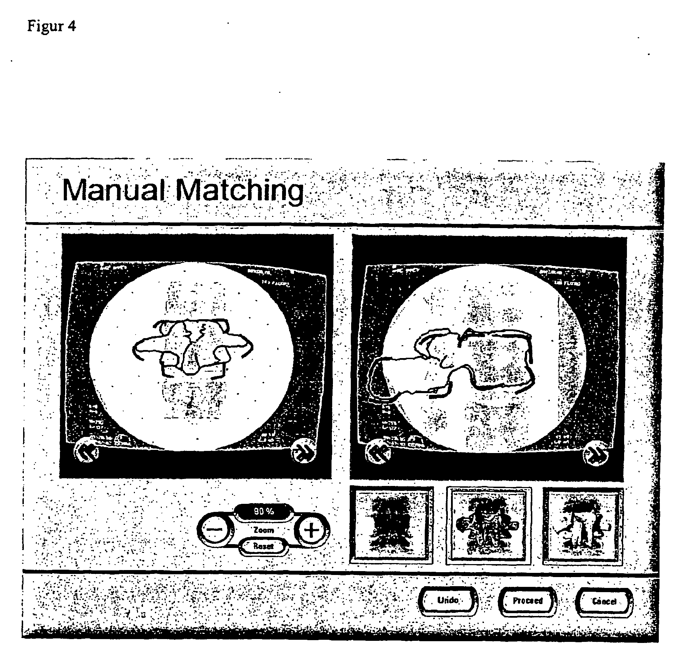

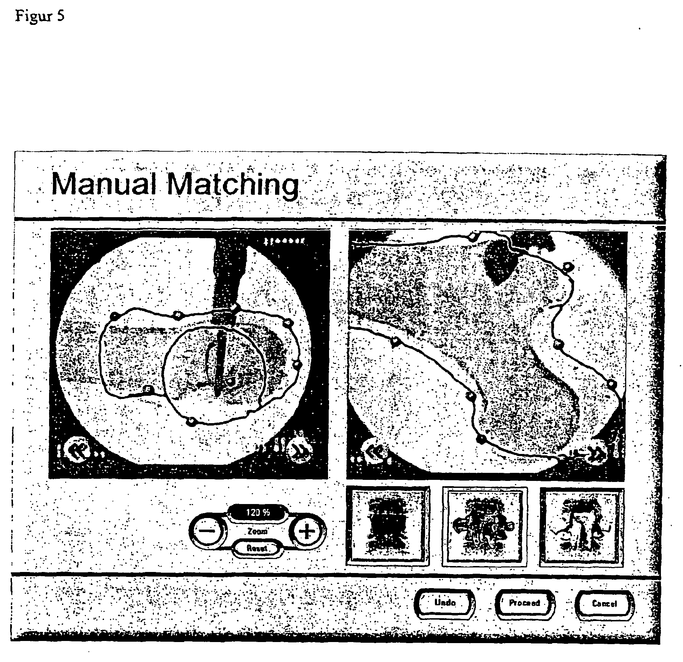

Die Figuren 2 bis 6 zeigen Screenshots einer Computeranwendung, die das erfindungsgemäße Verfahren unterstützt. Es sind jeweils Bildschirmansichten für die zweidimensionale Überlagerung von Röntgenaufnahmen und Projektionen aus generischen Modelldaten erkennbar, mit hervorgehobenen Landmarken des Modells, die vom Bediener auf die entsprechenden Stellen der Patienten-Röntgenaufnahmen gesetzt werden, um so die geeignete Transformation zu ermitteln.

Die Figur 2 zeigt zwei Ansichten für ein Hüftmodell, die Figuren 3 und 4 für ein Wirbelkörpermodell, wobei nur die Landmarken angezeigt sind, und die Figur 5 zeigt Femur-Ansichten.

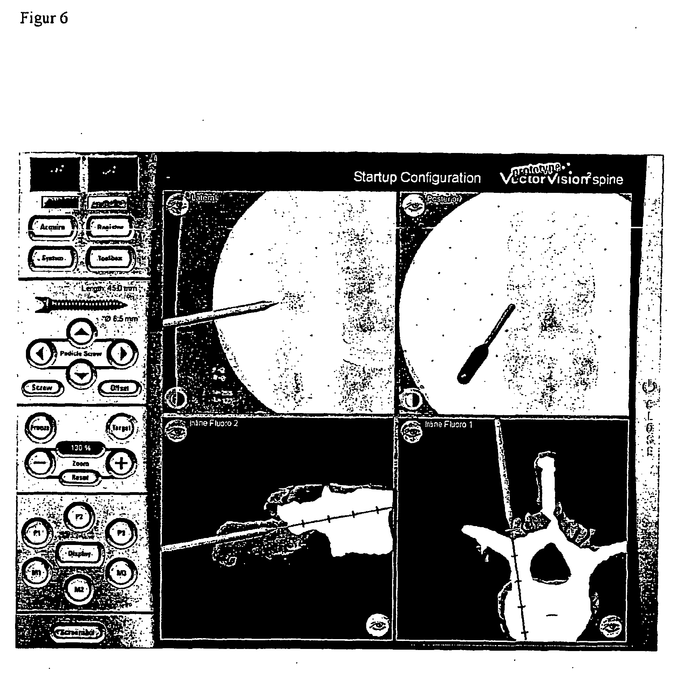

Wenn die Transformationsvorschrift dann ermittelt und das generische Modell angepasst ist, kann, wie in Figur 6 gezeigt, eine Fluoro-Navigation erfolgen, und zwar auf den Röntgenbildern (oben) und unterstützt durch angepasste generische 3D-Bilddarstellungen (unten).

## Patentansprüche

1. Verfahren zur computergestützten medizinischen Navigation bzw. präoperativen Behandlungsplanung bei dem mittels einer Positionserfassungseinheit die aktuelle Position eines Patienten oder eines Patientenkörperteils sowie die Positionen von medizinischen Behandlungsgeräten oder behandlungsunterstützenden Geräten erfasst werden, und bei dem die erfassten Positionsdaten Körperstrukturdaten zugeordnet werden, um die Körperstrukturdaten in Zuordnung zu den Positionsdaten gemeinsam im Rahmen der Behandlungsunterstützung zu verwenden, **dadurch gekennzeichnet, dass** Körperstrukturdaten verwendet werden, die auf der Basis eines dreidimensionalen generischen Modells erhalten werden, wobei das Modell durch eine Datenverknüpfung auf zweidimensionaler Ebene mit patientencharakteristischen, zweidimensionalen Erfassungsdaten angepasst wird.

2. Verfahren nach Anspruch 1, bei dem die Körperstrukturdaten in der Form eines Bilddatensatzes bereitgestellt werden, insbesondere als Schichtbilderfassungs-Datensatz.

3. Verfahren nach Anspruch 1 oder 2, bei dem das generische Modell eines oder mehrere der folgenden Merkmale aufweist:
- es umfasst eine typische bzw. durchschnittliche Körperstruktur;
- es umfasst ein statistisches Modell der Körperstruktur, insbesondere basierend auf statistischen Auswertungen einer unbestimmten Anzahl von Bilddatensätzen;
- es umfasst eine Vielzahl von Körperstrukturen gleicher Art.
- es umfasst einen zwei- oder dreidimensionalen Datensatz einer Körperstruktur umfasst, insbesondere auch ein geometrisches Modell.

4. Verfahren nach einem der Ansprüche 1 bis 3, bei dem die patientencharakteristischen Daten Diagnosedaten sind, die vom Patienten erhalten werden, nämlich Daten der folgenden Art, auch in Kombination:
- Röntgenbilddaten aus vorab oder während der Behandlung erstellten Röntgenbildern, insbesondere zwei- oder multiplanaren Röntgenbildern,
- Computertomographie- oder Kernspintomographie-Bilddaten,
- digital rekonstruierte Röntgenbilddaten (DRRs = digitally reconstructed radiographs),
- akquirierte Punkt-Positionsinformationen der Patienten-Körperstruktur, insbesondere von natürlichen oder künstlichen Landmarken,
- Daten über Größe, Gewicht oder Körperabschnitts- bzw. Gliedmaßenlängen des Patienten.

5. Verfahren nach einem der Ansprüche 1 bis 4, bei dem die Anpassung des generischen Modells durch eine oder mehrere der folgenden Methoden erfolgt:
- manuelle Anpassung mit Hilfe einer Bilddarstellungsunterstützung, insbesondere durch das Versetzen von Punkten und Landmarken oder das Verschieben, Verdrehen, Dehnen, oder Komprimieren des generischen Modells auf einer Bildschirmausgabe mittels Benutzerinterface-Einrichtungen,
- automatische Bildfusionsverfahren, die insbesondere auf der automatischen Erkennung bestimmter anatomischer Merkmale basieren,
- Bilddaten des generischen Modells, insbesondere digital rekonstruierte Röntgenbilder, und solche aus Computertomographie- oder Kemspintomographie-Bilddatensätzen werden in Deckung gebracht bzw. fusioniert,
worauf die angepassten Körperstrukturdaten computergestützt errechnet werden.

6. Verfahren nach einem der Ansprüche 1 bis 5, bei dem die Positionsdaten, die bei der Ermittlung der patientencharakteristischen Erfassungsdaten erhalten werden, insbesondere durch die Akquirierung von Landmarkenpositionen oder durch im Navigationssystem registrierte Röntgenbildaufnahmen verwendet werden, um die Registrierung der angepassten Körperstrukturdaten im Navigationssystem vorzunehmen und Behandlungsgeräte bzw. behandlungsunterstützende Geräte in Registrierung zur angepassten Körperstruktur bildlich darzustellen oder einzusetzen.

7. Verfahren nach einem der Ansprüche 1 bis 6, bei dem das dreidimensionale generische Modell mit patienteneigenen Röntgenbilder überlagert, und eine Projektion des Modells den jeweiligen Röntgenbildern angepasst wird.

8. Verfahren nach Anspruch 7, bei dem in den patienteneigenen Bilddaten die Projektion anatomischer Landmarken oder Geometrien, automatisch oder manuell erkannt und die Projektion von Modellstrukturen an diese zweidimensional erkannten Landmarken angepasst wird.

9. Verfahren nach Anspruch 8, bei dem die Anpassung mittels einer Transformationsvorschrift durchgeführt wird, die es ermöglicht auch die dem Modell hinterlegte Information, beispielsweise einen CT oder MR-Datensatz, entsprechend zu modifizieren, so dass ein aus Schichtaufnahmen bestehender Datensatz des Patienten für die Navigation Verwendung finden kann.

10. Verfahren nach Anspruch 9, bei dem der Patientendatensatz über eine Projektionsberechnung als Digital Reconstructed Radiography (DRR) dargestellt und zur automatischen oder manuellen Verifikation des Modells mit den patientenspezifischen Daten verglichen wird.

11. Verfahren nach einem der Ansprüche 1 bis 10, bei dem die Anpassung anhand einer Überlagerung patientenspezifischen Röntgenbilder, welche ein zweidimensionales Summationsbild aus einer definierten Projektionsrichtung darstellen, und einer Projektion des dreidimensionalen generischen Modells auf dieses Summationsbild erfolgt.

12. Verfahren nach einem der Ansprüche 1 bis 11, bei dem eine für das Modell gewonnene Verformungs- und Rotationsvorschrift auf die dem Modell hinterliegende Information angewendet wird, um einen dreidimensionalen Bilddatensatz zu erzeugen oder einen schon vorhandenen Bilddatensatz mit Hilfe dieser Vorschrift zu verformen.

13. Programm, das, wenn es auf einem Computer läuft oder in einem Computer geladen ist, den Computer veranlasst, ein Verfahren gemäß einem der Ansprüche 1 bis 12 durchzuführen.

14. Computerprogramm-Speichermedium, das ein Programm nach Anspruch 13 aufweist.

## Claims

1. A method for computer-assisted medical navigation and/or pre-operative treatment planning, wherein the current position of a patient or a part of a patient's body and the positions of medical treatment devices or treatment-assisting devices are detected by means of a position detection unit, and wherein said detected positional data are assigned to body structure data, in order to jointly use said body structure data in assignment with said positional data, within the context of assisting the treatment, **characterised in that** body structure data are used which are obtained based on a three-dimensional generic model, wherein said model is adapted by two-dimensional data linking with two-dimensional, patient-characteristic detection data.

2. The method as set forth in claim 1, wherein said body structure data are provided in the form of an image data set, in particular as a tomographic imaging data set.

3. The method as set forth in claim 1 or 2, wherein said generic model comprises one or more of the following features:
- it includes a typical or average body structure;
- it includes a statistical model of said body structure, in particular based on statistical evaluations of an indefinite number of image data sets;
- it includes a multitude of body structures of the same type;
- it includes a two- or three-dimensional data set of a body structure, and in particular a geometric model.

4. The method as set forth in any one of claims 1 to 3, wherein said patient-characteristic data are diagnostic data obtained from the patient, namely data of the following type, also in combination:
- x-ray image data from x-ray images produced before or during treatment, in particular bi-planar or multi-planar x-ray images;
- computer tomography or nuclear spin tomography image data;
- digitally reconstructed x-ray image data (DRRs = digitally reconstructed radiographs);
- acquired point-positional information of the patient's body structure, in particular of natural or artificial landmarks;
- data on the size, weight or lengths of the body section or of one or more limbs of the patient.

5. The method as set forth in any one of claims 1 to 4, wherein said generic model is adapted using one or more of the following methods:
- manually adapting with the assistance of image representation, in particular by offsetting points and landmarks or by shifting, rotating, stretching or compressing said generic model on a screen output by means of user-interface means;
- automatic image fusion methods, in particular based on automatically identifying particular anatomical features;
- registering and/or fusing image data of said generic model, in particular digitally reconstructed x-ray images, and the same from computer tomography or nuclear spin tomography image data sets;
after which the adapted body structure data are calculated with computer assistance.

6. The method as set forth in any one of claims 1 to 5, wherein said positional data obtained while determining said patient-characteristic detection data, in particular by acquiring landmark positions or by x-ray imaging registered in said navigation system, are used to register said adapted body structure data in said navigation system and to visually display or introduce treatment devices and/or treatment-assisting devices, registered with respect to the adapted body structure.

7. The method as set forth in any one of claims 1 to 6, wherein the three-dimensional generic model is superimposed with patient-specific x-ray images, and a projection of the model is adapted to the respective x-ray images.

8. The method as set forth in claim 7, wherein projecting anatomical landmarks or geometries in the patient-specific image data is automatically or manually identified, and projecting model structures is adapted to these two-dimensionally identified landmarks.

9. The method as set forth in claim 8, wherein the model is adapted by means of a transformation guideline which also enables the information stored in the model, for example a CT or MR data set, to be appropriately modified, such that a data set of the patient consisting of tomographic images can be used for navigation.

10. The method as set forth in claim 9, wherein by calculating the projection, the patient data set is displayed as digital reconstructed radiography (DRR), and compared with the patient-specific data in order to automatically or manually verify the model.

11. The method as set forth in any one of claims 1 to 10, wherein the image data set is adapted by superimposing patient-specific x-ray images representing a two-dimensional summation image from a defmed direction of projection, and projecting the three-dimensional generic model onto said summation image.

12. The method as set forth in any one of claims I to 11, wherein a deforming and rotating guideline obtained for the model is applied to the information stored in the model, to generate a three-dimensional image data set or to deform an already existing image data set with the aid of said guideline.

13. A program which, when running on a computer or loaded on a computer, causes said computer to perform a method in accordance with any one of claims 1 to 12.

14. A computer program storage medium comprising a program as set forth in claim 13.

## Revendications

1. Procédé pour la navigation médicale ou la planification de traitement préopératoire assistées par ordinateur dans lequel la position actuelle d'un patient ou d'une partie de corps de patient ainsi que les positions d'appareils de traitement médicaux ou d'appareils assistant le traitement sont enregistrés au moyen d'une unité d'enregistrement de position, et dans lequel les données de position enregistrées sont attribuées à des données de structure du corps, afin d'utiliser les données de structure du corps en attribution aux données de position conjointement dans le cadre de l'assistance du traitement, **caractérisé en ce qu'**on utilise des données de structure du corps qui sont obtenues sur la base d'un modèle générique en trois dimension, le modèle étant adapté par un enchaînement de données sur un plan en deux dimensions avec des données d'enregistrement en deux dimensions et caractéristiques du patient.

2. Procédé selon la revendication 1, dans lequel les données de structure du corps sont mises à disposition sous la forme d'un ensemble de données d'image, en particulier sous la forme d'un ensemble de données pour l'enregistrement d'image de couche.

3. Procédé selon la revendication 1 ou 2, dans lequel le modèle générique présente une ou plusieurs des caractéristiques suivantes :
- il comprend une structure de corps typique et moyenne ;
- il comprend un modèle de statistique de la structure du corps, en particulier basé sur des analyses statistiques d'un nombre indéfini d'ensembles de données d'image ;
- il comprend un grand nombre de structures de corps de même type ;
- il comprend un ensemble de données à deux ou trois dimensions d'une structure de corps, en particulier également un modèle géométrique.

4. Procédé selon l'une quelconque des revendications 1 à 3, dans lequel les données caractéristiques du patient sont des données de diagnostic qui sont obtenues du patient, à savoir des données du type suivant, également en combinaison :
- des données de radiographie à partir de radios effectuées auparavant ou pendant le traitement, en particulier de radiographies biplanaires ou multiplanaires,
- des données d'image de tomographie informatisée ou de tomographie à spin nucléaire,
- des données de radiographie reconstituées numériquement (DRRs = Digitally Reconstructed Radiographs),
- des informations de position de point acquises de la structure du corps du patient, en particulier de repères naturels ou artificiels,
- des données concernant la taille, le poids ou les longueurs de partie de corps ou de membres du patient.

5. Procédé selon l'une quelconque des revendications 1 à 4, dans lequel l'adaptation du modèle générique est effectuée par une ou plusieurs des méthodes suivantes :
- adaptation manuelle à l'aide d'une assistance de représentation en image, en particulier par le déplacement de points et de repères ou le décalage, la rotation, l'allongement ou la compression du modèle générique sur une sortie d'écran au moyen de dispositifs d'interface utilisateur,
- procédés automatiques de diffusion d'image, qui sont basés en particulier sur la reconnaissance automatique de caractéristiques anatomiques définies,
- données d'image du modèle générique, en particulier des radiographies reconstituées de façon numérique et des données d'image provenant d'ensembles de données d'image de tomographie informatisée ou de tomographie à spin nucléaire sont amenées en recouvrement ou fusionnées, après quoi les données adaptées de structure du corps sont calculées de façon automatisée.

6. Procédé selon l'une quelconque des revendications 1 à 5, dans lequel les données de position, qui sont obtenues lors de la détermination des données d'enregistrement caractéristiques du patient, en particulier par l'acquisition de positions de repères ou par des radiographies enregistrées dans le système de navigation sont utilisées pour effectuer l'enregistrement des données adaptées de structure du corps dans le système de navigation et de représenter en image ou d'utiliser des appareils de traitement ou des appareils d'assistance de traitement d'enregistrement pour la structure de corps adaptée.

7. Procédé selon l'une quelconque des revendications 1 à 6, dans lequel le modèle générique en trois dimensions est superposé avec des radiographies propres du patient, et une projection du modèle est adaptée aux radiographies concernées.

8. Procédé selon la revendication 7, dans lequel, dans les données d'image propres du patient, la projection de repères ou géométries anatomiques est détectée automatiquement ou manuellement et la projection de structures de modèle est adaptée à ces repères identifiés en deux dimensions.

9. Procédé selon la revendication 8, dans lequel l'adaptation est effectuée au moyen d'une prescription de transformation, qui permet de modifier en conséquence également l'information déposée dans le modèle, par exemple un ensemble de données CT ou MR, de sorte qu'un ensemble de données, composé de prises de vue dé couche, du patient peut être utilisé pour la navigation.

10. Procédé selon la revendication 9, dans lequel l'ensemble de données du patient est représenté au moyen d'un calcul de projection sous la forme de Digitally Reconstructed Radiographs (DRR) et comparé pour la vérification automatique ou manuelle du modèle avec les données spécifiques du patient.

11. Procédé selon l'une quelconque des revendications 1 à 10, dans lequel l'adaptation s'effectue à l'aide d'une superposition de radiographies spécifiques du patient, qui représentent une image cumulée en deux dimensions à partir d'un sens de projection défini, et d'une projection du modèle générique en trois dimensions sur cette image cumulée.

12. Procédé selon l'une quelconque des revendications 1 à 11, dans lequel une prescription de déformation et de rotation obtenue pour le modèle est appliquée à l'information à la base du modèle, afin de générer un ensemble de données d'image en trois dimensions ou de déformer un ensemble de données d'images existant à l'aide de cette prescription.

13. Programme qui, lorsqu'il fonctionne sur un ordinateur ou est chargé dans un ordinateur, demande à l'ordinateur d'appliquer un procédé selon l'une quelconque des revendications 1 à 12.

14. Support de mémorisation de programme informatique, qui présente un programme selon la revendication 13.
